# EUROPÄISCHE PATENTANMELDUNG

(11) **EP 4 091 715 A1**
(43) Veröffentlichungstag der Anmeldung: **23.11.2022**
(21) Anmeldenummer: 22167956.6
(22) Anmeldetag: 12.04.2022
(51) Int. Cl.: B01L 3/00, B01L 9/06

(54) **TESTSTREIFENANORDNUNG MIT BEHÄLTERN**

(30) Priorität: 29.04.2021 DE 102021111102
(71) Anmelder: Aeneas GmbH & Co. KG, 55234 Wendelsheim (DE)
(72) Erfinder: Matthias, Dr. Torsten, 55237 Flonheim (DE)
(74) Vertreter: Schaafhausen Patentanwälte PartGmbB

(57) **Zusammenfassung**

Die Erfindung betrifft ein Teststreifensystem mit Behältern sowie geeignete Träger hierfür. Die Teststreifenanordnung ist geeignet, um verschiedene Arten von chemischen, biologischen oder biochemischen Tests durchzuführen und mit einem geeigneten Analysegerät auszuwerten, wobei die Teststreifenanordnung einen ersten Bereich mit mindestens einem Behälter und einen zweiten Bereich mit ebenfalls mindestens einem Behälter aufweist; wobei der Behälter des ersten Bereichs einen ebenen durchsichtigen Boden aufweist und der Behälter des zweiten Bereichs einen V-förmigen oder U-förmigen Boden aufweist.

## Beschreibung

Die Erfindung bezieht sich auf ein Teststreifensystem mit Behältern sowie geeignete Träger hierfür. Die Teststreifenanordnung ist geeignet, um verschiedene Arten von chemischen, biologischen oder biochemischen Tests durchzuführen und mit einem geeigneten Analysegerät auszuwerten.

Aus dem Stand der Technik ist eine Vielzahl an solchen Teststreifensystemen bekannt. Die meisten dieser Teststreifensysteme haben jedoch eine einheitliche Form, d. h. es ist eine einheitlich ausgebildete Anzahl von Behältern vorhanden, welche hintereinander angeordnet sind und meist durch kleine Stege oder Brücken miteinander verbunden sind. Solche Anordnungen finden häufig Verwendung im analytischen und diagnostischen Labor, u.a. in PCR Polymerase-Kettenreaktionssystemen oder werden als Reaktionsbehälter für den Nachweis von ELISAs verwendet. Hierbei sind die Streifensysteme u.a. weiß oder schwarz eingefärbt oder durchsichtig, so dass durch Reagenzien angeregte Reaktionen oder auch durch Licht oder sonstige Strahlung angeregte Reaktionen innerhalb eines Behälters des Teststreifens sichtbar gemacht werden können oder beispielsweise mittels geeigneter Filter analysiert werden können. Die Behälter, welche ein solches Teststreifensystem beinhaltet, sind dabei meist von gleicher geometrischer Bauart. Meist weisen die Behälter hierbei eine konische und halbrunde Form im Bereich des Bodens auf oder sind im Bereich des Bodens vollkommen eben und haben einer zylinderförmigen Ausgestaltung. Eine konische und halbrunde Form hat den Vorteil, beim Mischen der Reagenzien bzw. beim Ansetzen eines Versuches wenig Probenvolumen oder auch Volumen der Testreagenzien zu verschwenden oder zu verlieren. Eine konische und halbrunde Form eignet sich jedoch nicht besonders gut zur Detektion, da Streulicht, Interferenzen oder ähnliche Phänomene im Bereich des konisch geformten Bodens auftreten können. Zudem werden meist auch die Behälterböden mit speziellen nicht biologischen oder biologischen Beschichtungen, wie beispielsweise Antikörpern oder Antigenen oder dergleichen versehen, was wiederum bei einer konischen Form unter Umständen schwieriger bis nahezu nicht möglich ist. Hinzu kommt, dass eine Auswertung beispielsweise von Fluoreszenzereignissen oder Farbumschlägen bei Beschichtung einer konischen Form schwierig bis unmöglich ist.

Die Erfindung hat daher zum Ziel Teststreifenanordnungen mit Kavitäten zu verbessern, vornehmlich solche, welche zur Analyse von Proben oder Reagenzien eingesetzt werden.

Hierbei soll insbesondere eine Anordnung zur Verfügung gestellt werden, in der die eingesetzten Reagenzien oder Proben möglichst ohne Verlust verarbeitet werden.

Eine weitere Aufgabe besteht darin, effiziente und qualitative Analysebedingungen zur Verfügung zu stellen. Hierbei soll insbesondere eine Effizienz in puncto Auswertbarkeit und Durchführung verschiedener Analysemethoden wie Kolorimetrie, Photometrie, Fluoreszenz, Chemilumineszenz, Elektrochemilumineszenz und dergleichen zur Verfügung gestellt werden.

Eine weitere Aufgabe besteht darin, eine Teststreifenanordnung mit Kavitäten zur Verfügung zu stellen, die eine einfache Handhabung erlaubt, insbesondere was die Durchführung der Versuche oder Tests im Hinblick auf Sicherheit, Verwechselbarkeit und erleichterte Handhabung angeht.

Eine weitere Aufgabe der Erfindung besteht auch darin, die Teststreifenanordnung als eine Art "ready-to-go" Versuchsanordnung zur Verfügung zu stellen, welche alle für einen Test notwendigen Reagenzien bevorratet und eine hohe Langzeitstabilität in punkto Lagerung aufweist.

Die Aufgaben werden durch eine Teststreifenanordnung mit Kavitäten sowie einen hierfür geeigneten Träger gemäß den unabhängigen Ansprüchen 1 und 10 gelöst. In den Unteransprüchen sind vorteilhafte Weiterbildungen und bevorzugte Ausführungen angegeben.

Die erfindungsgemäße Teststreifenanordnung mit Kavitäten umfasst dabei einen ersten Bereich mit mindestens einem Behälter und einen zweiten Bereich mit ebenfalls mindestens einem Behälter. Hierbei ist es so, dass der Behälter des ersten Bereiches einen ebenen durchsichtigen Boden aufweist und der Behälter des zweiten Bereichs einen vorwiegend V-förmigen oder vorwiegend U-förmigen Boden aufweist. Der Behälter des ersten Bereiches wird im Sinne der Erfindung auch Reaktions- oder Verdünnungsbehälter genannt. In dem Behälter des zweiten Bereichs kann ein Reagens appliziert werden oder liegt in dieser bereits vor, mit welchem ein Test durchgeführt werden kann. Bei komplexen Tests kann die erfindungsgemäße Teststreifenanordnung mit Behältern auch weitere Behälter im zweiten Bereich aufweisen. Je nachdem, um was für einen Test es sich handelt, können dann gegebenenfalls verschiedene Reagenzien aus den verschiedenen Behältern des zweiten Bereichs nacheinander vermischt werden, um dann einen Test durchzuführen. Hierbei kann gegebenenfalls ein Behälter des zweiten Bereichs eine Pufferlösung beinhalten, ein weiterer Behälter des zweiten Bereichs kann dann spezielle Markierungsmittel oder andere Reagenzien usw. beinhalten. Als Behälter kann im Sinne der Erfindung auch eine Vertiefung oder Kavität verstanden werden, die in der Lage ist, ein Fluid, vorwiegend ein Liquid aufzunehmen und zumindest vorübergehend aufzubewahren.

Durch die erfindungsgemäße Bodenform des Behälters des zweiten Bereiches lässt sich hierbei der Inhalt des Behälters nahezu vollständig aufnehmen, wodurch nahezu keine Rückstände verbleiben und somit eine Sicherheit der Durchführung des Tests gegeben ist.

Durch die erfindungsgemäße Bodenform des Behälters des ersten Bereiches, welche eben und durchsichtig ist, lässt sich eine Analyse der Reagenzien bzw. Proben mittels geeigneter Analysegeräte gut und reproduzierbar durchführen. Geeignete Analysemethoden, die hierbei angewendet werden können, sind beispielsweise kolorimetrische, photometrische, spektrometrische und lichtmikroskopische Methoden, ELISAs, Kolorimetrie, Photometrie, Fluoreszenz, Chemilumineszenz, Elektrochemilumineszenz und andere Methoden.

Gemäß einer vorteilhaften Ausführungsform der Teststreifenanordnung sind zur Sicherheit vor Kontaminationen oder Verdampfung oder zur Sicherheit vor Verschüttung die mit Reagenzien versehenen Behälter des zweiten Bereichs mit einer Folie abgedeckt und verschlossen.

Gemäß einer weiteren vorteilhaften Ausführungsform der Teststreifenanordnung mit Behältern ist die Folie, mit welcher der zweite Bereich bedeckt ist, derart gefertigt, dass diese Folie mit einer Pipettenspitze durchstochen werden kann. Weiter ist die Folie aus einem Material gefertigt, was zur Versiegelung der Teststreifen geeignet ist. Hierbei kann die Folie durch ein geeignetes Material bzw. durch eine geeignete Methode fest mit dem Teststreifen verbunden werden. Geeignete Materialien wären beispielsweise ein Adhäsiv, Kleber oder Polymer, mit einer großen Affinität zum Teststreifenmaterial und einer geringen Dampfdurchlässigkeit, welcher für eine stabile Langzeitlagerung geeignet ist, oder eine Methode wie Verschweißen mit Hitze oder Licht. Die Folie kann einschichtig oder mehrschichtig sein und mit einem Trägermaterial, wie z.B. einem Polymer, Leichtmetall oder anderen Materialien ausgestattet sein. Das Folienmaterial weist dabei ebenfalls bevorzugt Eigenschaften auf, welche die Dichtigkeit und Haltbarkeit sowie Stabilität der Folie und der Reagenzien generell gewährleisten.

Gemäß einer vorteilhaften Ausführungsform der erfindungsgemäßen Teststreifenanordnung mit Behältern weisen die Behälter des ersten und zweiten Bereichs einen Rand auf. Erfindungsgemäß ist es so, dass der Rand des zweiten Bereichs höher ist als der des ersten Bereiches. Hierbei ist vorteilhafterweise der Rand des zweiten Bereiches um 0,2 mm höher als der Rand des ersten Bereiches. Bevorzugterweise ist dabei der Rand des zweiten Bereiches um 0,4 mm höher als der Rand des ersten Bereiches und besonders bevorzugt ist der Rand 0,6 mm höher als der des ersten Bereiches. Eine vorteilhafte Differenz zwischen dem Rand des ersten Bereiches und dem Rand des zweiten Bereiches hat sich bei einem 0,5 mm höheren Rand des zweiten Bereiches gezeigt. Durch den etwas höheren Rand des zweiten Bereiches gegenüber dem ersten Bereich wird ein deutlich verbessertes Verschweißen bzw. Verkleben der Folie mit der Teststreifenanordnung möglich. Hierdurch kann eine bessere Dichtigkeit für längere Haltbarkeiten und Stabilitäten gewährleistet werden. Hinzu kommt hier auch noch die hohe Dampfbarriere der gesamten Teststreifenanordnung sowie des Folienmaterials, damit die Reagenzien auch lange Zeit stabil gelagert werden können.

Gemäß einer weiteren vorteilhaften Ausführungsform der erfindungsgemäßen Teststreifenanordnung mit Behältern ist diese mit Ausnahme des Bodens des ersten Bereiches aus einem lichtdichten, lichtdurchlässigen oder durchsichtigen Material gefertigt. Materialien, welche hierfür hauptsächlich verwendet werden, sind Thermoplaste wie Polyethylen, Polypropen, Polyamid, Polymethylmethacrylat oder Polystyrol. Auch sind Duroplaste hierfür geeignet wie beispielsweise Melami-Formaldehyd-Harz (Phenoplaste) oder UF Aminoplaste. Es ist jedoch auch denkbar, Glas oder Glassilikate sowie auch Keramik hierfür einzusetzen.

Gemäß einer weiteren vorteilhaften Ausführungsform wird ein Cyclo-olefine Polymer (COP) bzw. ein Cyclo-olefine Copolymer (COC) verwendet, wobei sich diese Materialien durch eine besondere Beständigkeit, hohe optische Transmission im visuellen Spektralbereich, geringe Eigenfluoreszenz, geringe Wasseraufnahme sowie geringe Dampfdurchlässigkeit auszeichnen.

Auch ist es möglich, dass die Behälter des ersten Bereiches aus einem Material gefertigt sind und die Kavitäten des zweiten Bereiches ein anderes Material aufweisen, je nachdem welche Versuchsbedingungen gerade vorherrschen sollen. Vorteilhafterweise können die Behälter des zweiten Bereiches ganz oder teilweise aus einem lichtdichten, lichtdurchlässigen oder durchsichtigen Material gefertigt sein.

Gemäß einer weiteren vorteilhaften Ausführungsform der erfindungsgemäßen Teststreifenanordnung mit Behältern weist diese einen Barcode oder eine Markierung zur Identifizierung der Teststreifenanordnung auf. Ein solcher Barcode oder eine Markierung kann beispielsweise ein Strichcode, QR-Code, RFID-Code oder sonst üblicher Barcode oder eine Markierung sein. Der Barcode enthält als Information unter anderem die Zusammensetzung der Reagenzien, welche in der Teststreifenanordnung vorliegen können und kann dadurch über die Haltbarkeit der Reagenzien Aufschluss geben, wenn diese bei einer bestimmten Temperatur und Bedingung gelagert werden. Weiter kann ein solcher Barcode die damit versehene Teststreifenanordnung eindeutig identifizieren, wodurch ein späteres Verwechseln oder Vertauschen von Testergebnissen verhindert werden soll. Darüber hinaus kann ein solcher Barcode leicht von einem dafür vorgesehenen Lesegerät erfasst und gelesen werden, so dass eine eindeutige Zuordnung der Teststreifenanordnungen beispielsweise zu einem bestimmten Patienten oder Test gewährleistet ist, wobei für Nicht-Zugangsberechtigte der Barcode aufgrund seiner Erscheinungsform nicht einem bestimmten Test oder einer bestimmten Person zugeordnet werden kann. Hierdurch kann eine anonymisierte Durchführung von Tests und deren weitere Aufbewahrung gewährleistet werden.

Gemäß einer weiteren vorteilhaften Ausführungsform der erfindungsgemäßen Teststreifenanordnung mit Kavitäten weist diese seitlich am unteren Ende der Teststreifenanordnung, genauer seitlich des Bodens des ersten und zweiten Bereichs, Führungsstege auf. Diese Führungsstege dienen dazu, dass der Teststreifen in einen dafür vorgesehenen Träger präzise positioniert und formschlüssig lösbar fixiert werden kann, um so beispielsweise das Pipettieren, Waschen und Auswerten der Tests und das Auswerfen des Teststreifen aus dem Träger zu ermöglichen.

Gemäß einer weiteren vorteilhaften Ausführungsform der erfindungsgemäßen Teststreifenanordnung mit Behältern verjüngt sich diese von oben her betrachtet vom ersten Bereich hin zum zweiten Bereich oder der zweite Bereich weist einen Abschnitt auf, in welchem keine Führungsstege verlaufen. Gemäß einer weiteren Ausgestaltung sind die Seitenflächen des zweiten Bereiches durchgehend mit Material versehen, wodurch Seitenflächen entstehen, die eine bessere Haptik der Teststreifenanordnung im zweiten Bereich gewährleisten. Weiter wird durch die zuvor beschriebenen Ausgestaltungen an der Teststreifenanordnung und einen an die Form der Teststreifenanordnung angepasste Trägerform erreicht, dass die Teststreifenanordnung nur in einer ganz bestimmten Richtung bzw. einer bestimmten Weise in einem Träger angeordnet werden kann. Hierdurch wird beispielsweise ein unabsichtliches Vertauschen des ersten mit dem zweiten Bereich verhindert, so dass ein falsches Einsetzen der Teststreifenanordnung beispielsweise in ein Analysegerät nicht stattfinden kann, wodurch Anwendungsfehler praktisch ausgeschlossen sind.

Gemäß einer weiteren vorteilhaften Ausführungsform der erfindungsgemäßen Teststreifenanordnung mit Behältern weist diese an einer Seite eine Klemmung mit Klemmfeder auf. Diese Klemmung mit Klemmfeder hat den Vorteil, dass die Teststreifenanordnung in einen dafür vorgesehenen Träger eingesetzt werden kann und darin sicher verbleibt. Diese Verbindung kann durch Drücken der Feder nahezu kraftfrei oder kraftarm durch ein Verschieben des Teststreifens im Träger wieder gelöst werden, wodurch die Teststreifenanordnung wieder vom dafür vorgesehenen Träger gelöst werden kann.

Gemäß einer weiteren vorteilhaften Ausführungsform der erfindungsgemäßen Teststreifenanordnung mit Behältern weist die Teststreifenanordnung mindestens ein Reagens in mindestens einem Behälter des zweiten Bereiches und/oder eine Beschichtung in mindestens einer Kavität des ersten Bereiches auf. Vorteilhafterweise ist hierbei die Beschichtung auf den Boden der Kavität beschränkt. Unter Beschichtung wird im Sinne der Erfindung auch eine Bedampfung, Bedruckung, Lackierung, Besprühung und/oder Inkubation verstanden. Im Sinne der Erfindung stellt die Beschichtung hierbei vorwiegend eine nicht biologische oder biologische Beschichtung dar, wie beispielsweise ein Antiköper, ein Antigen, eine Probe wie beispielsweise Blut oder Urin, eine bestimmte chemische Substanz, eine bestimmte biologische Substanz DNA oder RNA verstanden. In einem beispielhaften Testszenario kann dann zum Beispiel eine Probe in den Behälter des ersten Bereiches appliziert werden. Hinzu kommen dann ein Reagenz oder mehrere Reagenzien der Behälter des zweiten Bereichs, wodurch eine Reaktion ausgelöst wird, welche mittels geeigneter Analyseinstrumente dann in der Kavität des ersten Bereiches analysiert werden kann.

Weiter umfasst die Erfindung auch einen Träger für eine zuvor beschriebene Teststreifenanordnung. Hierbei ist der erfindungsgemäße Träger für eine Teststreifenanordnung mit Behältern derart ausgebildet, dass die Teststreifenanordnung innerhalb des Trägers mittels einer Klemmung und/oder mittels Führungsstegen, welche in dafür vorgesehene Führungsschienen des Trägers geschoben werden, wieder lösbar fixiert werden. Erfindungsgemäß kann die Teststreifenanordnung hierbei mit der Klemmung mit Klemmfeder in den Träger eingebracht werden und durch eine leichte Kraft auf die Feder am Träger fixiert werden. Jedoch ist es auch wieder möglich, durch eine leichte Kraft auf die Feder die Teststreifenanordnung wieder vom Träger zu lösen. Erfindungsgemäß kann der Träger jedoch auch derart ausgebildet sein, dass die Teststreifenanordnung in den Träger geschoben wird, wobei dieser hierfür geeignete Führungsschienen bzw. Aussparungen aufweist.

Gemäß einer vorteilhaften Ausführungsform des erfindungsgemäßen Trägers für eine Teststreifenanordnung weist dieser eine zusätzliche Feder auf, wodurch die Teststreifenanordnung, welche mittels der Stege in dafür vorgesehene Führungsschienen des Trägers eingebracht wird, zusätzlich wieder lösbar am Träger fixiert wird. Eine solche Feder kann beispielsweise durch ein leicht gebogenes Metallband ausgebildet sein. Hierdurch wird eine leichte Kraft auf eine Seite der Teststreifenanordnung ausgeübt. Die Kraft ist gerade so groß, um die Teststreifenanordnung präzise und stabil im Träger zu halten. Die Kraft ist jedoch nur so groß, dass immer noch ein Verschieben der Teststreifenanordnung für die Zuführung oder Entnahme für den Anwender möglich ist.

Gemäß einer weiteren vorteilhaften Ausführungsform des erfindungsgemäßen Trägers für eine Teststreifenanordnung ist der Träger in Form eines Rechtecks, eines Voll- oder Halbkreises oder eines Kreissegments ausgebildet. Prinzipiell kann der Träger in jeglicher geometrischer Form ausgebildet sein mit der Maßgabe, dass der Träger eine für die Analyse durch ein Analysegerät geeignete Form aufweist.

Gemäß einer weiteren vorteilhaften Ausführungsform des erfindungsgemäßen Trägers für eine Teststreifenanordnung weist der Träger eine geeignete komplementäre Form auf, um in einem dafür geeigneten Analysegerät als Trägereinheit zu funktionieren.

Nachfolgend wird die erfindungsgemäße Teststreifenanordnung mit Behältern, sowie der erfindungsgemäße Träger, für eine solche Teststreifenanordnung in Ausführungsbeispielen und in Zeichnungen näher beschrieben. Die Erläuterungen sind lediglich beispielhaft und schränken den allgemeinen Erfindungsgedanken nicht ein.

Es zeigen:
- FIG. 1: eine schematische Darstellung einer Teststreifenanordnung 10 gemäß Stand der Technik in perspektivischer Ansicht;
- FIG. 2a: eine schematische Darstellung einer Ausführungsform der erfindungsgemäßen Teststreifenanordnung 10 in perspektivischer Ansicht;
- FIG. 2b: eine schematische Darstellung einer Ausführungsform der erfindungsgemäßen Teststreifenanordnung 10 in perspektivischer Ansicht mit Folie;
- FIG. 3a: eine schematische Darstellung einer Ausführungsform der erfindungsgemäßen Teststreifenanordnung 10 in perspektivischer Ansicht;
- FIG. 3b: eine schematische Darstellung einer Ausführungsform der erfindungsgemäßen Teststreifenanordnung 10 in Frontansicht;
- FIG. 3c: eine schematische Darstellung einer Ausführungsform der erfindungsgemäßen Teststreifenanordnung 10 in Rückansicht;
- FIG. 3d: eine schematische Darstellung einer Ausführungsform der erfindungsgemäßen Teststreifenanordnung 10 von unten;
- FIG. 4a: eine schematische Darstellung einer Ausführungsform der erfindungsgemäßen Teststreifenanordnung 10 aus FIG. 3 in Seitenansicht;
- FIG. 4b: eine schematische Darstellung in seitlicher Ansicht einer Schnittebene der Ausführungsform der erfindungsgemäßen Teststreifenanordnung 10 aus FIG. 4a;
- FIG. 5a: eine schematische Darstellung in Aufsicht einer Ausführungsform der erfindungsgemäßen Teststreifenanordnung 10 innerhalb einer Ausführungsform eines erfindungsgemäßen Trägers;
- FIG. 5b: eine schematische Darstellung in Aufsicht einer Ausführungsform der erfindungsgemäßen Teststreifenanordnung 10 mit Folie innerhalb einer Ausführungsform eines erfindungsgemäßen Trägers;
- FIG. 6: eine schematische Darstellung einer Ausführungsform eines erfindungsgemäßen Trägers ohne Teststreifenanordnung 10;
- FIG. 7: eine schematische Darstellung einer Ausführungsform eines erfindungsgemäßen Trägers mit eingesetzten Teststreifenanordnung 10 mit Folie 80;
- FIG. 8a-c: schematische Darstellungen der Abfolge des Einsetzens einer erfindungsgemäßen Teststreifenanordnung 10 in eine Ausführungsform eines erfindungsgemäßen Trägers;
- FIG. 9a-b: weitere schematische Darstellungen einer weiteren Möglichkeit, den erfindungsgemäßen Träger aus FIG. 8 mit einer Teststreifenanordnung 10 ebenfalls aus FIG. 8 zu bestücken.

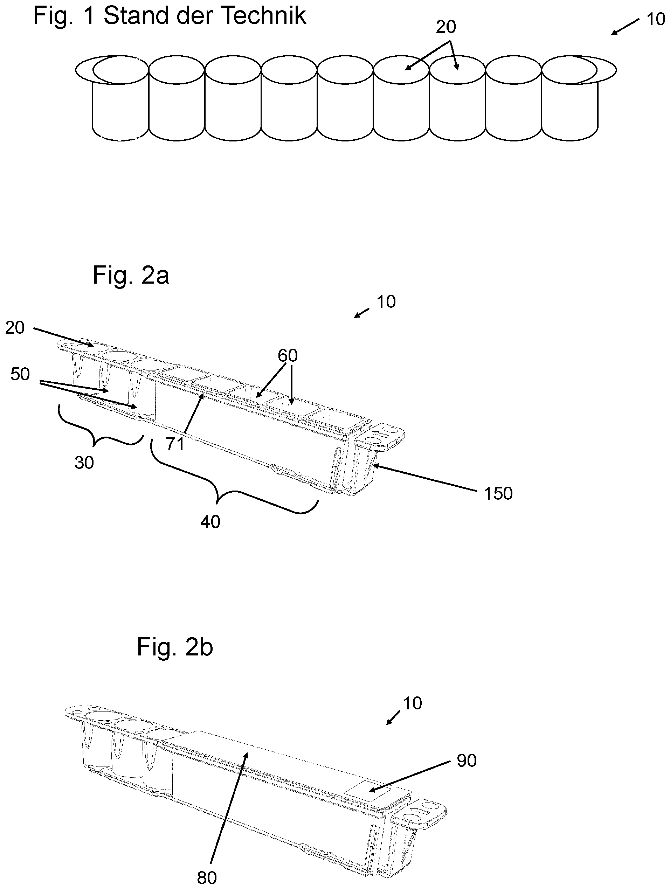

In FIG. 1 ist eine Teststreifenanordnung 10 gemäß Stand der Technik zu sehen. Hierbei ist deutlich, dass alle Behälter 20 der Teststreifenanordnung 10 einen flachen Boden aufweisen. Weiter kann dieser Teststreifen auch in eine dafür vorgesehene Halterung eingesetzt werden, jedoch liegt dieser dann nur lose an. Ein weiterer Nachteil dieser Teststreifenanordnung 10 gemäß Stand der Technik ist der, dass der Teststreifen verkehrtherum in den Träger eingesetzt werden kann und somit ein Vertauschen der Probe möglich ist bzw. die Zuordnung der Probe zu einem bestimmten Patienten oder Bezugswert so vertauscht werden kann. Ein weiterer Nachteil ist der, dass die Teststreifenanordnung 10 gemäß Stand der Technik nicht wieder lösbar fixiert in einem Träger befestigt werden kann. So kann zum Beispiel ein Ausklopfen von Luftblasen, welche die Nachweisreaktion stören könnten, manuell oder automatisch nicht erfolgen.

Die Figur 2a zeigt in einer perspektivischen schematischen Darstellung eine Ausführungsform der erfindungsgemäßen Teststreifenanordnung 10. Die dargestellte Teststreifenanordnung 10 weist ein transparentes durchsichtiges Material auf. Die Materialien können je nach Anwendungsfall gewählt werden, so dass beispielsweise bei einem schwarzen Material vermehrt Streulicht oder Interferenzen verringert werden, während andere Materialien wiederum andere Eigenschaften aufweisen.

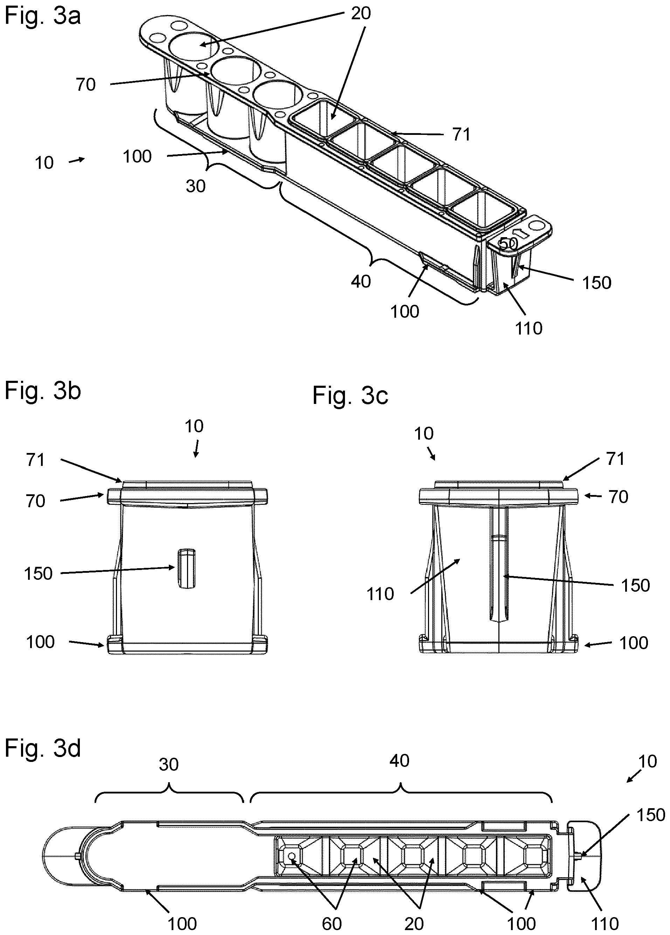

Weiter zu sehen sind auch Klemmfedern 150 zur Ausbildung einer Klemmung mit einem dafür geeigneten erfindungsgemäßen Träger. Des Weiteren sind auch in FIG. 3a die Führungsstege 100 zu erkennen, welche ebenfalls dazu dienen, die erfindungsgemäße Teststreifenanordnung 10 in einen geeigneten Träger zu führen und zu fixieren. Weiter zu erkennen in FIG. 2a ist auch ein erster Bereich 30 mit eher zylinderförmigen Behältern 20 sowie ein zweiter Bereich 40 mit eher in Quaderform gehaltenen Behältern 20. Darüber hinaus kann man erkennen, dass der zweite Bereich 40 einen höheren Rand 71 als der erste Bereich aufweist.

In der Figur 2b ist die bereits aus der Figur 2a bekannte erfindungsgemäße Teststreifenanordnung 10 mit einer Folie 80 dargestellt. Die Abdeckfolie 80 ist dabei auf dem etwas höheren Rand 71 der Behälter 20 des zweiten Bereiches appliziert. Die Folie 80, auch Siegelfolie genannt, ist dabei so konzipiert, dass sie mittels Pipettenspitzen leicht durchstochen werden kann. Weiter weist die Folie 80 eine Beschichtung auf, die so konzipiert ist, dass sie abbrandarm ist, und strukturiert, beschriftet oder bedruckt werden kann. Die Folie 80 ermöglicht es auch, die Teststreifenanordnung 10 als eine Art Lagerbehältnis mit gleichzeitig integrierter Testvorrichtung zu verwenden. Hierfür werden ein Reagenz oder mehrere Reagenzien in ein oder mehrere Behälter 20 des zweiten Bereichs 40 eingefüllt und mit oben genannter Folie 80 verschlossen. Dies kann zum Beispiel durch Verschweißen oder Verkleben geschehen. Durch den überstehenden bevorzugten 0,5 mm hohen Rand 71 der Kavitäten 20 des zweiten Bereichs 40 wird ein deutlich besseres Befestigen der Abdeckfolie 80 mit der Teststreifenanordnung 10 erzielt als dies herkömmlich der Fall ist. Hiermit kann eine bessere Dichtigkeit für längere Haltbarkeiten und Stabilitäten der in der Teststreifenanordnung 10 gelagerten Reagenzien gewährleistet und erzielt werden. Hinzu kommt eine hohe Dampfbarriere des Teststreifen- und Folienmaterials, wodurch die Reagenzien langzeitstabil gelagert werden können. Die Kavitäten 20 des ersten Bereiches 30, auch Reaktions- oder Verdünnungskavitäten 20 genannt, können in einem weiteren Produktionsschritt mit einem produktspezifischen Antigen, Antikörper oder anderen Proteinen oder Chemikalien beschichtet werden. Wird beispielsweise eine solche Reaktionskavität oder ein Reaktionsbehälter mit einem produktspezifischen Antigen beschichtet, kann ein spezifischer beispielsweise im Patientenserum befindlicher Antikörper nachgewiesen werden. Hierbei können jedoch nicht nur Patientenseren, sondern auch andere Analyten wie beispielsweise Urin oder CSF (eng. *cerebrospinal fluid*) eingesetzt werden. Zudem weist in diesem Beispiel die Folie 80 einen Barcode 90 auf, der eine eindeutige und dabei anonymisierte Zuordnung von Test und Probe sicherstellt.

In der FIG. 3a ist eine Ausführungsform der Erfindung gezeigt, in der der erste Bereich 30 mit drei Behältern 20 und der zweite Bereich 40 mit fünf Behältern 20 gut zu erkennen ist. Die Behälter 20 des ersten Bereiches 30 haben hierbei eine zylinderförmige Ausgestaltung, während die Behälter 20 des zweiten Bereiches 40 eine eher quaderförmige Ausgestaltung zeigen. Zu erkennen ist auch die Klemmfeder 110 mit Nase 150, die der zeitweisen Fixierung der Teststreifenanordnung 10 in einem Träger dient. Die Seitenflächen im zweiten Bereich 40 sind flächig ausgestaltet, was eine leichtere und bequemere Handhabung für den Benutzer, beispielsweise beim Einsetzen der Anordnung in einen Träger, ermöglicht. FIG. 3b und FIG. 3c zeigen die Front- bzw. Rückansicht einer Ausführungsform der erfindungsgemäßen Teststreifenanordnung 10. Gut zu erkennen sind die Nasen 150, die zur Fixierung im Träger dienen, sowie der erhöhte Rand 71 zur Applikation einer Folie 80 im zweiten Bereich 40. In FIG. 3d ist eine Ansicht von unten auf eine Ausführungsform der erfindungsgemäßen Teststreifenanordnung 10 abgebildet. Zu erkennen sind hier vor allem die Führungsstege 100 am Boden im ersten Bereich 30 sowie im zweiten Bereich 40.

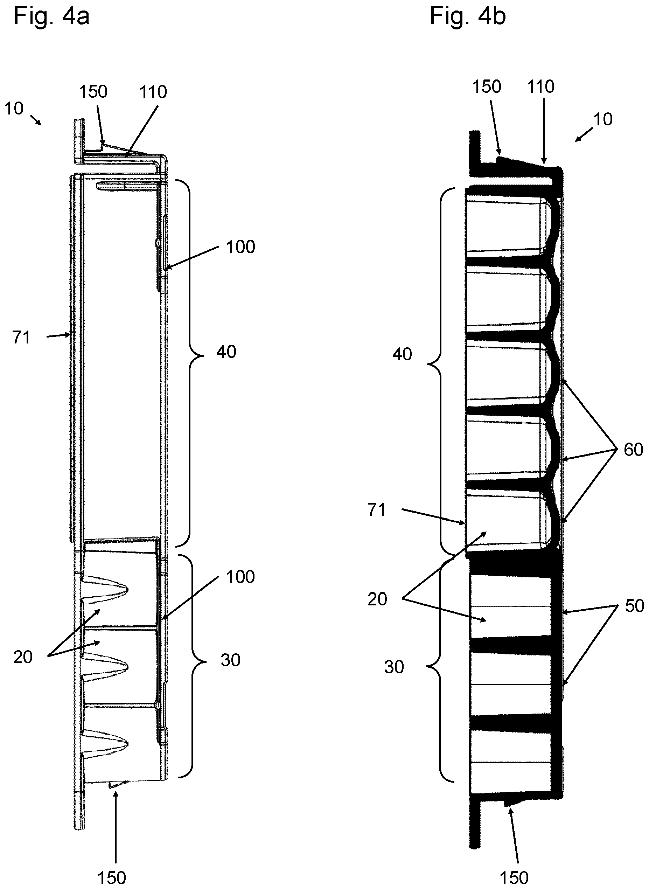

In FIG. 4a ist eine schematische Darstellung einer Ausführungsform der erfindungsgemäßen Teststreifenanordnung 10 in Seitenansicht zu sehen. Gut erkennen kann man die Klemmung mit Klemmfeder 100, welche darüber hinaus eine kleine, aus dem Material der Teststreifenanordnung 10 gebildete Nase 150 aufweist. An der gegenüberliegenden Seite der Klemmfeder 100 ist ebenfalls eine Nase 150 zu sehen. Diese Nasen 150 ermöglichen zusammen mit dem ebenfalls gegenüberliegenden Henkeln eine sichere Fixierung der erfindungsgemäßen Teststreifenanordnung 10 in einem dafür geeigneten Träger 120. Ebenfalls zu sehen ist der leicht erhöhte Rand 71 der Kavitäten 20 im zweiten Bereich 40 im Gegensatz zu den Kavitäten 20 des ersten Bereiches 30.

In FIG. 4b ist eine Ausführungsform der erfindungsgemäßen Teststreifenanordnung 10 aus FIG. 4a in Darstellung eines Längsschnittes zu sehen, welcher mittig, von oben gesehen, von vorne nach hinten durch die Teststreifenanordnung 10 verläuft. Zu sehen ist, dass die Kavitäten 20 des ersten Bereiches 30 einen flachen Boden 50 aufweisen, während die Kavitäten 20 des zweiten Bereiches 40 einen eher V-förmigen oder auch U-förmigen Boden 60 aufweisen. Durch diese Bodenform der Kavitäten 20 des zweiten Bereiches 40 bleibt bei der Entnahme von Flüssigkeiten weniger Totvolumen zurück, wodurch weniger Testreagenzien oder andere Flüssigkeiten eingesetzt werden müssen, was wiederum die allgemeinen Kosten reduziert. Weiter ist die die Klemmung mit Klemmfeder 110 zu erkennen mit den kleinen aus dem Material der Teststreifenanordnung 10 gebildeten Nasen 150.

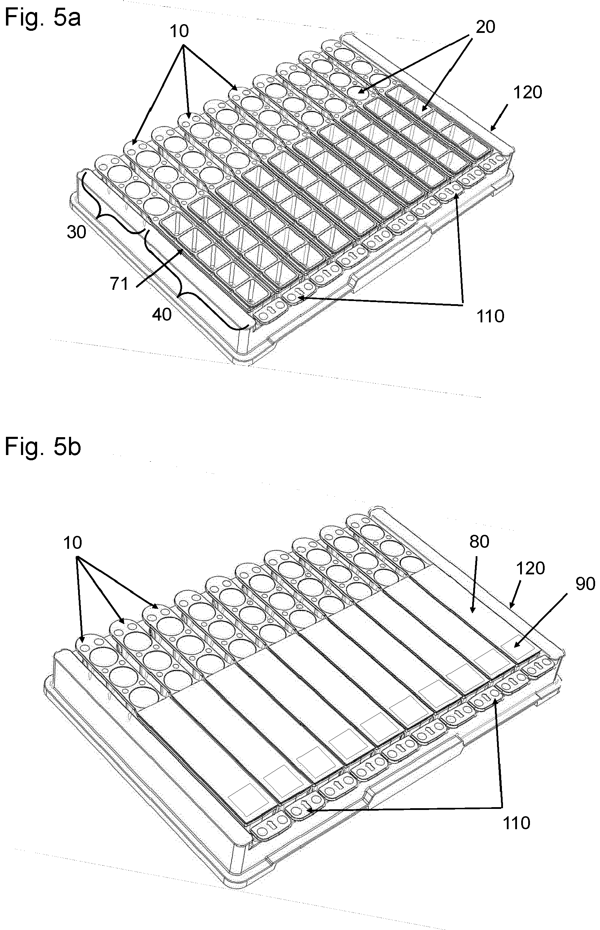

FIG. 5a und 5b zeigt eine schematische Darstellung von einem erfindungsgemäßen Träger 120, welcher mit Ausführungsformen einer erfindungsgemäßen Teststreifenanordnung 10 bestückt ist, einmal mit Folien 80 (FIG. 5b) und ohne Folien (FIG. 5a). Vorteil einer solchen Träger-Teststreifen-Anordnungskombination ist, dass sich die einzelnen Teststreifenanordnungen 10 leicht aus dem Träger 120 lösen lassen, die anderen Teststreifenanordnungen 10 jedoch ortsfest im Träger 120 verbleiben können.

Wie beispielsweise auch in FIG. 3 zu sehen ist, verjüngt sich die Teststreifenanordnung 10 im ersten Bereich 30 verglichen zum zweiten Bereich 40 hin etwas. Diese Verjüngung ermöglicht ein fehlerfreies Einsetzen der Teststreifenanordnung 10 im späteren dafür vorgesehenen Träger 120 und bietet somit ein hohes Maß an Gebrauchstauglichkeit. Die Teststreifenanordnung 10 kann aufgrund dieser Form nur in einer Richtung in den hierfür vorgesehenen erfindungsgemäßen Trägern 120, auch *Frame* genannt, eingesetzt werden. Ein falsches Handling wird damit von vornherein vermieden. Der Endanwender entnimmt eine Teststreifenanordnung 10 beispielsweise mit vorbefüllten Reagenzien und/oder beschichteten Reaktions- oder Verdünnungsbehältern 20 aus einem dafür vorgesehenen Verpackungsmaterial und setzt diese Teststreifenanordnungen 10 beispielsweise von vorne oder von oben in einen dafür vorgesehenen Träger 120 ein. Anschließend wird ein solcher erfindungsgemäßer Träger 120 mit der erfindungsgemäßen Teststreifenanordnung 10 in einem Analysegerät positioniert und der Test automatisch oder teilautomatisch prozessiert.

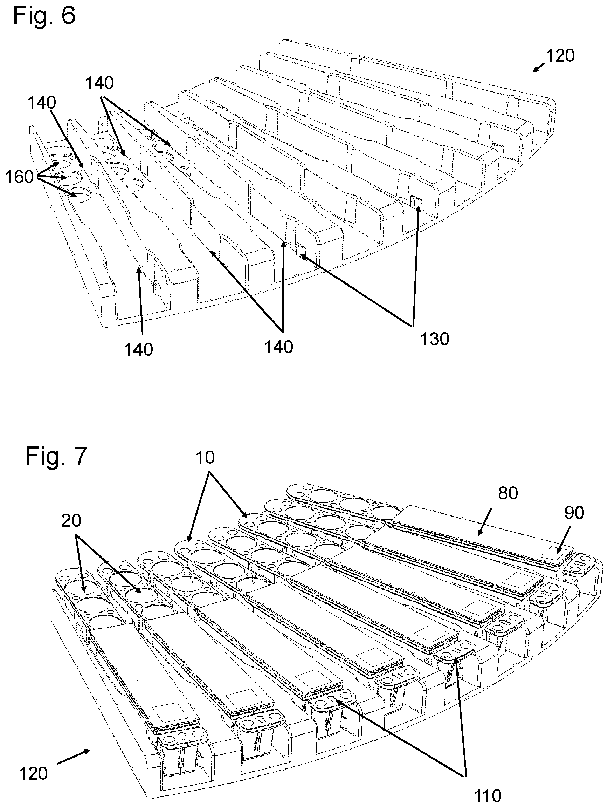

In FIG. 6 ist beispielsweise eine schematische Darstellung einer Ausführungsform eines erfindungsgemäßen Trägers 120 in einer Kreisabschnittsform ohne erfindungsgemäße Teststreifenanordnungen 10 zu sehen. Gut zu erkennen ist hierbei die zusätzliche Feder 130 des Trägers, welche die Teststreifenanordnung 10 durch eine Gegenkraft wieder lösbar fixieren kann. Ebenfalls zu sehen sind die Führungsschienen 140 bzw. Aussparungen, in welchen die Führungsstege 100 der Teststreifenanordnungen 10 eingebracht werden und so zusätzlich ein Herausrutschen nach oben der Teststreifenanordnungen 10 aus dem Träger 120 verhindern. Weiter zu sehen ist, dass der Träger 120 in dem Bereich, in dem später der erste Bereich 30 der Teststreifenanordnung 10 Platz findet, Aussparungen in Form von Durchgangsbohrungen 160 aufweist. Diese Durchgangsbohrungen 160 sind dafür vorgesehen, die Analyse der Tests oder Testreaktionen zu gewährleisten.

In FIG. 7 ist eine schematische Darstellung einer Ausführungsform eines erfindungsgemäßen Trägers 120 mit eingesetzten Teststreifenanordnungen 10 zu sehen. Auch in dieser Ausführungsform kann jede einzelne Teststreifenanordnung 10 separat vom Träger 120 gelöst werden, ohne dass hierdurch eine Beeinträchtigung der anderen Teststreifenanordnungen 10 besteht.

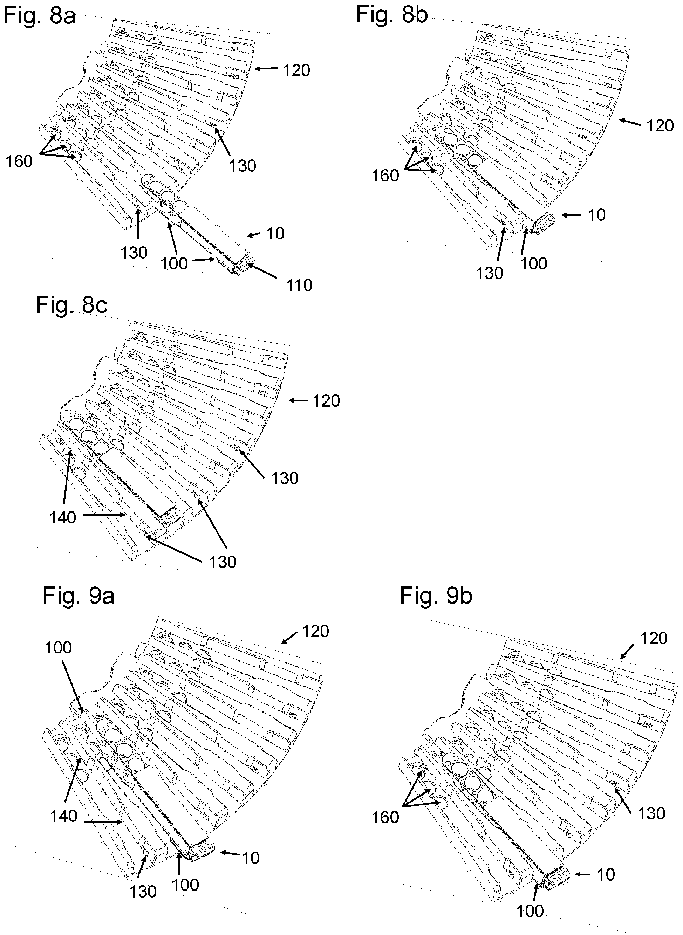

In den schematischen Abfolge-Darstellungen FIG. 8a-c ist das Einsetzen einer erfindungsgemäßen Teststreifenanordnung 10 in einer Ausführungsform eines erfindungsgemäßen Trägers 120 dargestellt. Hierbei wird die Teststreifenanordnung 10 seitlich in den Träger 120 geschoben (FIG. 8a). Mittels der Führungsstege 100 und der dafür vorgesehenen Führungsschienen 140 bzw. Aussparungen wird die Teststreifenanordnung 10 sicher im Träger fixiert. Durch die Verjüngung der Teststreifenanordnung 10 im ersten Bereich 30 sowie eine hierzu komplementäre Verjüngung des Trägers 120 kann die Teststreifenanordnung 10 auch nur in einer Richtung ganz in den Träger 120 eingeschoben werden. Durch die zusätzliche Feder 130, welche im Träger 120 verbaut ist, wird die Teststreifenanordnung 10 vor dem Herausrutschen geschützt. Diese Klemmverbindung lässt sich jedoch durch den Endanwender wieder kraftarm lösen, so dass die Teststreifenanordnung 10 wieder aus dem Träger 120 entnommen werden kann.

FIG. 9a sowie 9b zeigen jeweils eine weitere schematische Darstellung einer Möglichkeit, den erfindungsgemäßen Träger 120 aus FIG. 8 mit einer Teststreifenanordnung 10 zu bestücken. Hierzu wird die Teststreifenanordnung 10 von oben her in den Träger 120 eingebracht. Durch die angesprochene Verjüngung im zweiten Bereich 40 der Teststreifenanordnung 10 und die komplementäre Form des Trägers 120 lässt sich die Teststreifenanordnung 10 nur in einer Richtung von oben her in den Träger einsetzen. Im letzten Schritt dieses Vorgangs wird wiederum die Teststreifenanordnung 10 kraftarm nach vorne geschoben, um so durch die am Träger 120 verbaute zusätzliche Feder 130 vor dem Herausrausrutschen geschützt zu werden. Durch die bodennahen Führungselemente wie die Führungsschiene 140 bzw. Aussparung des Trägers 120 sowie die Stege 100 der Teststreifenanordnung 10 ist eine präzise Führung und Positionierung der Teststreifenanordnung 10 während des Pipettierens, Waschens, Auswertens und des Auswerfens der Teststreifenanordnung 10 bzw. der darin befindlichen Tests möglich. Die zusätzlichen Federn oder Federklemmelemente 110 ermöglichen eine einfache Montage, Entnahme und Zuführung der gesamten Teststreifenanordnung 10. Ebenfalls kann durch die am Träger 120 vorgesehene Feder 130 ein für den Anwender hör- und spürbares Einrasten der Teststreifenanordnung 10 im Träger 120 erzielt werden, um sicherzustellen, dass die Teststreifenanordnung 10 korrekt in den Träger 120 eingesetzt wurde.

### Bezugszeichen

- 10: Teststreifenanordnung
- 20: Behälter
- 30: erster Bereich
- 40: zweiter Bereich
- 50: durchsichtiger Boden
- 60: V- oder U-förmiger Boden
- 70: Rand
- 71: Rand des zweiten Bereiches
- 80: Folie
- 90: Barcode
- 100: Führungsstege
- 110: Klemmung mit Klemmfeder
- 120: Träger
- 130: Feder
- 140: Führungsschiene
- 150: Nase
- 160: Durchgangsbohrung

## Patentansprüche

1. Teststreifenanordnung (10) mit Behältern (20), wobei die Teststreifenanordnung (10) einen ersten Bereich (30) mit mindestens einem Behälter und einen zweiten Bereich (40) mit ebenfalls mindestens einem Behälter (20) aufweist; wobei der Behälter (20) des ersten Bereichs (30) einen ebenen durchsichtigen Boden (50) aufweist und der Behälter (20) des zweiten Bereichs einen V-förmigen oder U-förmigen Boden (60) aufweist.

2. Teststreifenanordnung (10) nach Anspruch 1, **dadurch gekennzeichnet, dass** der Behälter (20) des ersten (30) und des zweiten Bereichs (40) einen Rand (70) aufweisen, wobei der Rand (71) des zweiten Bereiches (40) höher ist als der des ersten Bereichs (30) und/oder wobei der Behälter (20) des zweiten Bereichs (40) mit einer Folie (80) verschlossen ist.

3. Teststreifenanordnung (10) nach einem der vorhergehenden Ansprüche, **dadurch gekennzeichnet, dass** die Teststreifenanordnung (10) mit Ausnahme des Bodens (50) des ersten Bereichs (30) aus einem lichtdichten, lichtdurchlässigen, oder durchsichtigen Material besteht.

4. Teststreifenanordnung (10) nach einem der vorhergehenden Ansprüche, **dadurch gekennzeichnet, dass** der zweite Bereich (40) ganz oder teilweise aus einem lichtdichten, lichtdurchlässigen, oder durchsichtigen Material besteht.

5. Teststreifenanordnung (10) nach einem der vorhergehenden Ansprüche, **dadurch gekennzeichnet, dass** die Teststreifenanordnung (10) oder die Folie (80) einen Barcode (90) zur Identifizierung der Teststreifenanordnung (10) aufweist.

6. Teststreifenanordnung (10) nach einem der vorhergehenden Ansprüche, **dadurch gekennzeichnet, dass** sie seitlich Führungsstege (100) aufweist.

7. Teststreifenanordnung (10) nach einem der vorhergehenden Ansprüche, **dadurch gekennzeichnet, dass** sich die Teststreifenanordnung (10) von oben betrachtet vom zweiten Bereich (40) hin zum ersten Bereich (30) verjüngt oder im zweiten Bereich (40) einen Abschnitt aufweist, in welchem keine Führungsstege (100) verlaufen.

8. Teststreifenanordnung (10) nach einem der vorhergehenden Ansprüche, **dadurch gekennzeichnet, dass** die Teststreifenanordnung (10) an einer Seite eine Klemmung mit Klemmfeder (110) aufweist und/oder eine Nase (150) aufweist.

9. Teststreifenanordnung (10) nach einem der vorhergehenden Ansprüche, **dadurch gekennzeichnet, dass** die Teststreifenanordnung (10) Testreagenzien aufweist, welche sich in den Behältern (20) des zweiten Bereichs (40) befinden und durch die Folie (80) verschlossen sind.

10. Teststreifenanordnung (10) nach einem der vorhergehenden Ansprüche, **dadurch gekennzeichnet, dass** die Folie (80) derart gefertigt ist, dass sie mit einer Pipettenspitze durchstochen werden kann.

11. Träger (120) für eine Teststreifenanordnung (10) nach den Ansprüchen 1 - 10, **dadurch gekennzeichnet, dass** die Teststreifenanordnung (10) mittels der Klemmung (110) oder der Führungsstege (100) der Teststreifenanordnung (10) die in dazu passende Führungsschienen (140) oder Aussparungen im Träger (120) eingebracht werden, wieder lösbar im Träger (120) fixiert wird.

12. Träger (120) nach Anspruch 11, **dadurch gekennzeichnet, dass** der Träger (120) zusätzlich eine Feder (130) aufweist, wodurch die Teststreifenanordnung (10), welche mittels der Führungsschienen (140) in den Träger (120) eingebracht wurden, zusätzlich wieder lösbar fixiert wird.

13. Träger (120) nach einem der Ansprüche 11 oder 12, **dadurch gekennzeichnet, dass** der Träger (120) in Form eines Rechtecks, eines Kreises oder eines Kreissegmentes ausgebildet ist.
